(19) Europäisches Patentamt
European Patent Office
Office européen des brevets

(11) Veröffentlichungsnummer: **0 144 894**
**A2**

(12) # EUROPÄISCHE PATENTANMELDUNG

(21) Anmeldenummer: **84114304.3**

(22) Anmeldetag: **27.11.84**

(51) Int. Cl.⁴: **C 07 H 15/04**
C 07 H 13/12, C 07 H 13/06
A 61 K 31/70

(30) Priorität: **07.12.83 DE 3344256**

(43) Veröffentlichungstag der Anmeldung:
**19.06.85 Patentblatt 85/25**

(84) Benannte Vertragsstaaten:
**BE CH DE FR GB IT LI**

(71) Anmelder: **BAYER AG**
**Konzernverwaltung RP Patentabteilung**
**D-5090 Leverkusen 1 Bayerwerk(DE)**

(72) Erfinder: **Krüger, Bernd-Wieland, Dr.**
**Sillerstrasse 49**
**D-5600 Wuppertal 11(DE)**

(72) Erfinder: **Lockhoff, Oswald, Dr.**
**Morgengraben 14**
**D-5000 Koeln 80(DE)**

(72) Erfinder: **Metzger, Karl Georg, Dr.**
**Pahlkestrasse 75**
**D-5600 Wuppertal 1(DE)**

(72) Erfinder: **Opitz, Hans-Georg, Dr. c/o Miles Lab. Inc.**
**2200 Powell Street P.O. Box 8817**
**Emeryville California 94662(US)**

(72) Erfinder: **Schaller, Klaus, Dr.**
**Am Sonnenschein 38**
**D-5600 Wuppertal 1(DE)**

(72) Erfinder: **Stünkel, Klaus Georg, Dr.**
**Am Eckbusch 55**
**D-5600 Wuppertal 1(DE)**

(72) Erfinder: **Zeiler, Hans-Joachim, Dr.**
**Elsbeeker Strasse 46**
**D-5620 Velbert 15(DE)**

(54) **Pharmazeutische Verwendung von substituierten O-Acyl-glycosiden.**

(57) Die Erfindung betrifft die pharmazeutische Verwendung von O-Acylglykosiden der allgemeinen Formel I

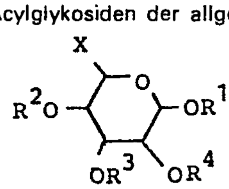

(I)

in welcher die Substituenten die in der Beschreibung angegebene Bedeutung haben.

EP 0 144 894 A2

BAYER AKTIENGESELLSCHAFT 5090 Leverkusen, Bayerwerk

Konzernverwaltung RP
Patentabteilung Dn/Hed/c

Pharmazeutische Verwendung von substituierten O-Acyl-glycosiden

Die Erfindung betrifft die pharmazeutische Verwendung von Verbindungen der allgemeinen Formel (I)

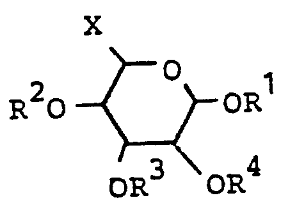

(I)

in welcher

X für Wasserstoff oder den Rest $CH_2OR^5$ steht,

$R^2, R^3, R^4, R^5$ gleich oder verschieden sind und für Wasserstoff oder den Rest

$-\overset{O}{\overset{\|}{C}}-Y-R^6$ stehen,

Y für Sauerstoff, Schwefel, NH, $CH_2$ steht,

$R^1$,$R^6$ gleich oder verschieden sind und für einen gegebenenfalls substituierten Kohlenwasserstoffrest mit bis zu 50 Kohlenstoffatomen steht mit der Maßgabe, daß mindestens einer der Reste $R^1$ und/oder $R^6$ zwischen 9 und 50 Kohlenstoffatome enthält.

Unter Kohlenwasserstoffrest in der Bedeutung der Reste $R^1$ und $R^6$, wird erfindungsgemäß ein geradkettiger oder verzweigter Alkylrest, ein geradkettiger oder verzweigter, ein- oder mehrfach ungesättigter Alkenylrest oder ein gesättigter oder ungesättigter alicyclischer Rest verstanden. Diese Bedeutungen können innerhalb desselben Restes $R^1$ und $R^6$ auch gemeinsam auftreten, d.h. beispielsweise als Alkylcycloalkyl, Alkenylcycloalkyl, etc.

In den Reste $R^1$ und $R^6$ können auch einzelne, im allgemeinen bis zu 5, vorzugsweise 1, 2 oder 3 Methylen- oder Methingruppen durch O, S und/oder N ersetzt sein. Bei einer Unterbrechung der Kette durch N trägt dieser Stickstoff entweder H oder einen $C_1$-$C_{20}$-Alkylrest oder einen -CO-Alkylrest, wobei diese Alkylgruppe 1 - 20 C-Atome aufweist.

Bevorzugt stehen $R^1$ und $R^6$ wahlweise für Alkyl oder Alkenylreste mit 1 bis 21 Kohlenstoffatomen, vorzugsweise mit 9 bis 21 C-Atomen.

Beispielhaft für gesättigte Reste seien hier genannt: Methyl, Ethyl, Propyl, i-Propyl, n-Butyl, i-Butyl, n-Pentyl, n-Hexyl, n-Heptyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-Octadecyl, n-Nonadecyl, Docosyl, Eicosyl, Tetracosyl, Triacontyl, Ethylpentyl, Methyldecyl, i-Propyldecyl, Methyltridecosyl, Pentahexadecyl, 1-Dodecylhexadecyl, 2-Dodecylhexadecyl, 3-Dodecylhexadecyl, 1-Hexadecyloctadecyl, 2-Hexadecyloctadecyl, 3-Hexadecyloctadecyl, 4-Hexadecyloctadecyl, 1-Octadecyleicosyl, 2-Octadecyleicosyl.

Ungesättigte Rest sind beispielsweise: Ethylen, Propenyl-1, Propenyl-2, i-Butenyl, Butenyl-1, Butenyl-2, 1-Pentenyl, 2-Pentenyl, 3-Pentenyl, 4-Pentenyl, 1-Hexenyl, 2-Hexenyl, 3-Hexenyl, 4-Hexenyl, 5-Hexenyl, 1-Decenyl, 5-Decenyl, 9-Decenyl, 8-Heptadecenyl, 1,3-Butadienyl, 1,3-Pentadienyl, 1,4-Pentadienyl, 2,4-Pentadienyl, 8,11-Heptadecandienyl, 8,11,14-Heptadecantrienyl. Im allgemeinen sind die längerkettigen ungesättigten Reste bevorzugt, speziell die ein- oder zweifach ungesättigten Alkenyle mit 9 - 21 C-Atomen. Die ungesättigten Kohlenwasserstoffreste können dabei als reine cis- oder trans-Isomere oder auch als Isomerengemische vorliegen.

Beispielhaft für Cycloalkyl seien genannt: Cyclopentyl, Cyclohexyl, Cyclododecyl.

Beispielhaft für Alkyl-Cycloalkylreste seien genannt: Methylcyclopentyl, Ethylcyclopentyl, n-Propylcyclopentyl, i-Propylcyclopentyl, n-Butylcyclopentyl, Octylcyclopentyl, Methylcyclohexyl, Ethylcyclohexyl, Propylcyclohexyl, Butylcyclohexyl, Hexylcyclohexyl, Decylcyclohexyl, Cyclopentylmethyl, Cyclopentylethyl, Cyclopentylpropyl, Cyclopentylbutyl, Cyclopentylpentyl, Cyclopentylhexyl, Cyclopentyloctyl, Cyclopentyldecyl, Cyclohexylmethyl, Cyclohexylethyl, Cyclohexylpropyl, Cyclohexylbutyl, Cyclohexylhexyl, Cyclohexyldecyl, Cyclopentylcyclohexylethyl, Cyclohexylcyclopentylethyl und Cyclohexylcyclohexylethyl.

Die Reste $R^1$, $R^6$ können substituiert sein, im allgemeinen 1-5-, vorzugsweise 1-3-fach. Als Substituenten kommen vorzugsweise die folgenden in Betracht: Halogen, vorzugsweise F, Cl oder Br, Amino, $C_1$-$C_6$-Alkylamino, Di-$C_1$-$C_6$-alkylamino, Oxo, OH, $C_1$-$C_6$-Alkoxy, SH, $C_1$-$C_6$-Alkylthio, $C_1$-$C_6$-Alkyl-COO und $C_1$-$C_6$-Alkyl-CO-NH.

Beispiele für Fälle in denen die Kohlenwasserstoffreste $R^1$, $R^6$ in Formel I durch O, S und N bzw. entsprechende Atomgruppierungen unterbrochen oder substituiert werden, sind Methoxyethyl, Ethoxyethyl, n-Propoxyethyl, n-Butoxyethyl, i-Propoxyethyl, i-Butoxyethyl, sek.-Butoxyethyl, Methoxyethoxyethyl, Ethoxyethoxyethyl, Propoxyethoxyethyl, i-Propoxyethoxyethyl, n-Butoxyethoxyethyl, i-Butoxyethoxyethyl, sek.-Butoxyethoxyethyl, Methoxyethoxyethoxyethyl, Ethoxyethoxyethoxy-

ethoxyethyl, n-Butoxyethoxyethoxyethyl, i-Butoxyethoxyethoxyethyl, sek.-Butoxyethoxyethoxyethyl, wenn Y für Sauerstoff, Schwefel, N-H oder $CH_2$ steht, Methoxyethoxy, Ethoxyethoxy, n-Propoxyethoxy, i-Propoxyethoxy, n-Butoxyethoxy, i-Butoxyethoxy, sek.-Butoxyethoxy, Methoxyethoxyethoxy, Ethoxyethoxyethoxy, n-Propoxyethoxyethoxy, i-Propoxyethoxyethoxy, n-Butoxyethoxyethoxy, i-Butoxyethoxyethoxy, sek.-Butoxyethoxyethoxy, wenn Y für $CH_2$ steht, Hydroxyheptadecenyl, Oxobutyl, Amino-decyl-, N-Methyl-aminodecyl-, Fluormethyl-, ß-Hydroxytridecyl- oder Mercaptoethylrest.

Die Verbindungen der Formel I enthalten mehrere chirale C-Atome und liegen als optische reine Diastereomere oder Diastereomerengemische vor.

Die erfindungsgemäß zu verwendenden Verbindungen der Formel I sind also O-(Cyclo)Alkylglycoside, die an einer oder mehreren Hydroxygruppen des Saccharidrestes mit einem Acyl-, Alkoxycarbonyl-, Alkylthiocarbonyl- oder einem Carbamoylrest versehen sind.

Besonders bevorzugt sind Verbindungen, die an der Hydroxygruppe in Position 6 als Saccharidreste mit einem Acyl-, Alkoxycarbonyl-, Alkylthiocarbonyl- oder einem Carbamoylrest versehen sind.

Die Verbindungen der Formel (I) sind teilweise bekannt (vgl. BE 873 132; US 3 597 417; US 3 628 928; B.Harlinova, et. al., Tenside Deterg. 1978, 72; A.T. Tulloch et. al., Canad. J. Chem., 46, 2485 (1968); E. Reinfeld et.al., Justus Liebigs Ann. Chem. 747, 39 (1971).

Die Verbindungen der Formel (I) können hergestellt werden indem man an sich bekannte oder nach bekannten Verfahren herstellbare O-Glycoside der Formel (II) (vgl. Methods in Carbohydrate Chemistry Academic Press, New York and London, 1972)

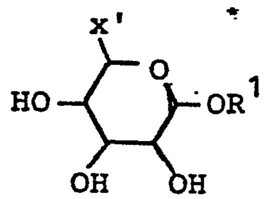

(II)

- wobei x' für H oder $CH_2OH$ steht und $R^1$ die obengenannte Bedeutung hat - entweder in freier, d.h. ungeschützter Form, oder in Form geschützter, gegebenenfalls aktivierter Derivate mit einem Carbonylderivat der Formel $Z-\overset{O}{\overset{\|}{C}}-Y-R^{6}$ , wobei $R^6$ und Y die obengenannte Bedeutung haben und Z für Halogen, vorzugsweise Chlor oder eine bei Acylierungsreaktionen gebräuchliche Abgangsgruppe, vorzugsweise einen aktivierenden Esterrest, oder eine Gruppe $O-OC-R^6$ steht, bzw. einem Isocyanat der Formel $R^6-NCO$, umsetzt, wobei man in einem organischen Lösungsmittel bzw. organisch-wäßrigen Lösungsmittel bei Temperaturen zwischen -80 und 160°C, vorzugsweise zwischen -20 und 60°C, gegebenenfalls in Gegenwart einer Base, arbeitet und nach beendeter Umsetzung das Reaktionsprodukt, gegebenenfalls nach Abspaltung eventuell vorhandener Schutzgruppen, in üblicher Weise aufarbeitet.

Als an sich bekannte Carbonylderivate $R^6-Y-\overset{O}{\overset{\parallel}{C}}-Z$ sind zu bevorzugen Anhydride, aktivierte Ester und Säurehalogenide, vorzugsweise Chloride.

Diese Verbindungen werden vorzugsweise in Gegenwart eines Verdünnungsmittels mit den O-Glycosiden umgesetzt, in welchem die Reaktionspartner vollständig oder auch nur teilweise gelöst sind.

Es kommen organische oder anorganische Solventien in Frage, vorzugsweise solche, die unter den Reaktionsbedingungen Nebenreaktionen möglichst herabsetzen oder verhindern.

Man kann sowohl in organischen Lösungsmitteln wie Ethern, z.B. Tetrahydrofuran und Dioxan oder Alkoholen, z.B. Ethanol und Propanol oder Ketonen, z.B. Aceton oder Methylethylketon, oder in Dimethylformamid, Essigester oder Pyridin als auch in Mischungen dieser Lösungsmittel untereinander und/oder mit Wasser arbeiten. Die Verwendung von wasserfreien Solventien ist im allgemeinen zu bevorzugen.

Die Säurederivate $R^6-Y-\overset{O}{\overset{\parallel}{C}}-Z$ bzw. Isocyanate $R^6-NCO$ werden in 1-10 Äquivalenten, bezogen auf O-Glycosid, eingesetzt.

Die Reaktionen können, vorzugsweise bei Verwendung von Säurehalogeniden und -anhydriden in Gegenwart basischer Hilfsstoffe durchgeführt werden. Verwendet werden können alle in der organischen Synthese üblichen basischen Verbindungen z.B. tertiäre aliphatische oder auch aromatische Amine oder aber Alkali- und Erdalkalihydroxide bzw. -carbonate wie Natronlauge, Natriumcarbonat oder Calciumcarbonat.

Das folgende Formelschema soll eine der bevorzugten Ausführungsformen der erfindungsgemäßen Darstellung von Verbindungen der Formel I beispielhaft erläutern:

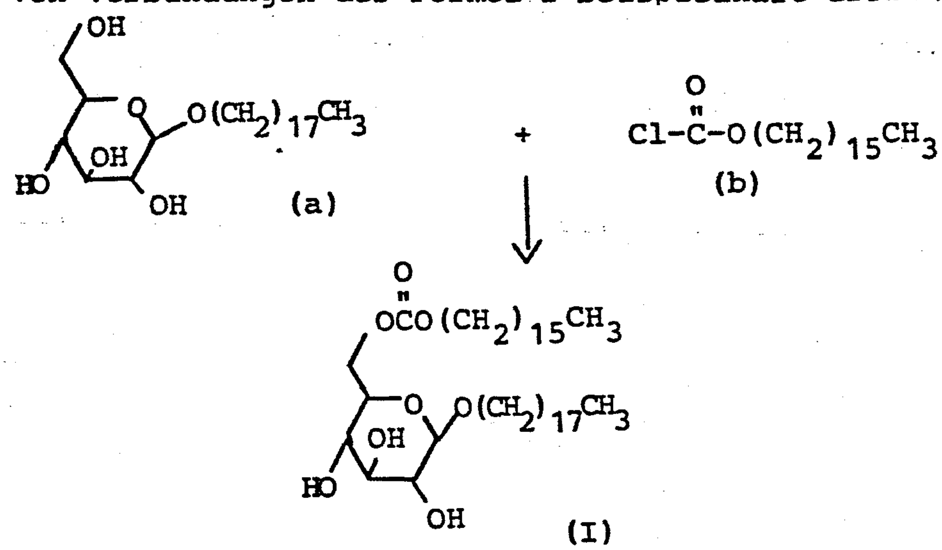

(a) (b) (I)

Stearyl-ß-D-glucopyranosid (a) wird mit Chlorameisensäure-hexadecyl-ester (b) zu Stearyl-6-0-Hexadecyloxycarbonyl-ß-D-glucopyranosid (I) umgesetzt.

Die vorliegende Verbindungsklasse weist eine Abwehrsteigernde Wirkung auf. Es wurde gefunden, daß die Verbindungsklasse die Antikörpersynthese des Immunsystems Antigen-spezifisch steigert und darüber hinaus die unspezifische wirteigene Abwehr verstärkt. Diese Ergebnisse wurden anhand der nachfolgenden Versuchsanordnungen erhalten.

Steigerung der primären humoralen Immunität in vivo gegen das lösliche Antigen Ovalbumin

NMRI-Mäuse wurden mit einer suboptimalen Antigendosis (1 µg/Tier, Tag 0) subcutan (s.c.) immunisiert. Bei suboptimaler Antigenstimulation wurde nur eine geringe Zahl von Lymphozyten der Tiere zur Antikörpersynthese angeregt. Die zusätzliche Behandlung der Tiere mit Verbindungen der genannten Beispiele der vorliegenden Erfindung ist in der Lage, bei einer einmaligen Applikation von 10 - 30 mg/kg den Antikörpertiter im Serum der Tiere signifikant zu steigern. Die Antikörpertiterbestimmung erfolgte durch indirekte Hämagglutination am Tag 10. Der Effekt der Behandlung wird durch den geometrischen Mittelwert der $\log_2$-Titer ausgedrückt.

Der immunstimulierende Effekt der genannten Verbindungen ist im Gegensatz zu anderen, z.B. bakteriellen Immunstimulantien wie LPS aus Gram-negativen Bakterien Antigenabhängig, d.h. die Substanzen bewirken überraschenderweise nur in Verbindung mit einem antigenen Reiz (hier Ovalbumin oder SE) die Induktion der Antikörpersynthese.

Sie haben im Gegensatz zu den erwähnten konventionellen Immunstimulatien keine mitogenen Eigenschaften.

Verträglichkeit und Möglichkeiten der Anwendung

Obwohl Verbindungen der beschriebenen Art ihre potenzierende Wirkung an der Maus beispielsweise bereits nach einer Einzeldosis von 10 mg/kg i.p. oder peroral entfalten, werden auch bei Applikation von 100 mg/kg keine toxischen Effekte beobachtet. Die genannten Stoffe verfügen deshalb über eine gute Verträglichkeit.

Die erfindungsgemäß zu verwendenden Verbindungen haben die Fähigkeit, einerseits bei Mischung mit einem Antigen dessen Immunität zu erhöhen, andererseits bei systemischer Applikation die immunologische Reaktivität des behandelten Organismus zu steigern. Dabei sind die genannten Stoffe in der Lage, die für die Antikörperbildung verantwortlichen Lymphozyten zu aktivieren.

Die Verbindungen der Formel I können somit als Adjuvantien in Mischung mit Impfstoffen dazu benutzt werden, den Impferfolg zu verbessern und den durch Immunität vermittelten Infektionsschutz gegenüber bakteriellen, viralen oder parasitären Erregern zu steigern.

Weiterhin eignen sich die Verbindungen der Formel I in Mischung mit verschiedensten Antigenen als Adjuvantien bei der experimentellen und industriellen Herstellung von Antiseren für Therapie und Diagnostik.

Darüber hinaus können die Verbindungen der Formel I auch ohne gleichzeitige Antigenzufuhr dazu benützt werden, bereits unterschwellig ablaufende Abwehrreaktionen bei Mensch und Tier zu fördern. Die Verbindungen eignen sich demnach besonders für die Stimulation der körpereigenen Abwehr, z.B. bei chronischen und akuten Infektionen oder bei selektiven (antigenspezifischen) immunologischen Defekten, sowie bei angeborenen, aber auch erworbenen allgemeinen (d.h. nicht antigenspezifischen) immunologischen Defektzuständen, wie sie im Alter, im Verlauf schwerer Primärerkrankungen und vor allem nach Therapie mit ionisierenden Strahlen oder mit immunsuppressiv wirkenden Stoffen auftreten. Die genannten Stoffe können somit vorzugsweise auch in Kombination mit antiinfektiösen Antibiotika, Chemotherapeutika oder anderen Heilverfahren verabreicht werden, um immunologischen Schädigungen entgegenzuwirken. Schließlich sind die beschriebenen Stoffe auch zur allgemeinen Prophylaxe von Infektionskrankheiten bei Mensch und Tier geeignet.

Die Verbindungen der Formel I erhöhen die Überlebensrate im Tiermodell der sytemischen Mäusecandidose und der akuten bakteriellen Infektion.

Versuchsbeschreibung

Mäuse vom Typ SPF-CFWI wurden intravenös mit $2\text{-}6 \times 10^5$ logarithmisch wachsenden Zellen von Candida albicans, suspendiert in physiologischer Kochsalzlösung, infiziert.

Beginnend mit dem 3. Tag post infektionem werden bei unbehandelten Kontrolltieren die ersten Krankheitssymptome erkennbar. Bis zum 5. Tag sterben die ersten Tiere an akuten Nierenversagen und bis zum 14. Tag post infectionem sind in der Regel mehr als 80 % der unbehandelten Tiere gestorben. In diesem Test sind die Verbindungen der Formel I krankheitsverzögernd wirksam. Eine signifikante krankheitsverzögernde Wirkung wurde erreicht, wenn die Substanz (Beispiel 1) jeweils 24 Stunden vor der Infektion in Konzentrationen von 1-50 mg/kg Körpergewicht intraperitoneal (i.p.) verabreicht wurde.

Bei behandelten Tieren wurde eine statistische signifikante Verlängerung der Überlebenszeit im Vergleich zu den unbehandelten Kontrollen beobachtet. Etwa 50 % der unbehandelten Tiere überlebten einen Beobachtungszeitraum von 14 Tagen, verglichen mit etwa 20 % unbehandelter Kontrolltiere.

Die Verbindungen der Formel I können allein als Prophylaktikum zur Abwehr bestehender Infektionen oder in Kombination mit einer antibiotischen Therapie zur Steigerung der therapeutischen Wirkung von Antibiotika und Chemotherapeutika (z.B. Penicilline, Cephalosporine, Aminoglykoside etc.) bei infizierten Menschen und Tieren verwendet werden.

Es wurde gefunden, daß Infektionen der Maus mit pathogenen Keimen, die innerhalb von 24-48 Stunden zum Tod

führen, durch eine prophylaktische Behandlung - bevorzugt intraperitoneal - mit 1-80 mg/kg der Verbindungen der Formel I therapiert werden können. Dies trifft für eine ganze Reihe grampositiver (z.B. Staphylokokken) und gramnegativer (z.B. E.coli, Klebsiella, Proteus, Pseudomonas) Krankheitserreger zu. Diese Aufzählung ist beispielhaft und keineswegs beschränkend aufzufassen. So überleben z.B. Mäuse, die mit dem pathogenen Stamm Klebsiella 63 infiziert worden waren, nach Behandlung (z.B. 18 Stunden vor Infektion) mit 10 - 40 mg/kg der Verbindung (Beispiel 1) zu 40 - 100 % diese Infektion, während von den unbehandelten Kontrolltieren nur 0 - 30 % überlebten.

In einem weiteren Versuchsmodell konnte gezeigt werden, daß die therapeutische Wirksamkeit von Antibiotika durch die Verbindungen der Formel I gesteigert werden kann. So wurden Mäuse mit dem Stamm Pseudomonas W. infiziert. Diese Infektion führte bei den meisten Kontrolltieren innerhalb 24 Stunden zum Tode. Eine weitere Gruppe wurde mit 4 mg/kg Sisomicin 30 Stunden post infektionem behandelt. Es konnte gezeigt werden, daß in der Versuchsgruppe, die mit den Verbindungen der Formel I 18 Stunden vor Infektion behandelt worden waren, die therapeutische Wirksamkeit des Sisomicins entscheidend verbessert werden konnte.

Die Erfindung betrifft auch pharmazeutischen Zubereitungen, die Verbindungen der Formel I enthalten. Die sind vorzugsweise Tabletten oder Gelatinekapseln, welche die

Wirkstoffe zusammen mit Verdünnungsmitteln, z.B. Laktose, Mannit, Sorbit, Cellulose und/oder Schmiermitteln, z.B. Kieselerde, Talk, Stearinsäure oder Salze davon, wie Magnesium- oder Calciumstearat, und/oder Polyethylenglykol, enthalten. Tabletten enthalten ebenfalls Bindemittel, z.B. Magnesiumsilikat, Stärken, wie Mais-, Weizen-, Reis- oder Pfeilwurzstärke, Gelatine, Traganth, Methylcellulose, Natriumcarboxymethylcellulose und/oder Polyvinylpyrrolidon, und wenn erwünscht Sprengmittel, z.B. Stärken, Agar, Alginsäure oder ein Salz davon, wie Natriumalginat und/oder Brausemischungen oder Adsorptionsmittel, Farbstoffe, Geschmacksstoffe und Süßmittel. Injizierbare Präparate sind vorzugsweise isotonische wäßrige Lösungen oder Suspensionen. Suppositorien, Salben oder Cremen sind in erster Linie Fettemulsionen oder -suspensionen. Die pharmazeutischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Druckes und/oder Puffer enthalten. Die erfindungsgemäßen pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren hergestellt und enthalten von etwa 0,1 % bis etwa 75 % insbesondere von etwa 1 % bis 50 % der genannten Aktivstoffe.

Oral applizierbare Präparate können auch mit einem gegen Magensaft beständigen Überzug versehen werden.

Die Verbindungen der Formel I können als abwehrsteigernde und immunpotenzierende Mittel zur Behandlung von chronischen und akuten Infektionen (z.B. bakterielle, virale und parasitäre) und malignen Tumoren verwendet werden. Sie können ebenfalls als Adjuvantien bei der Vakzinierung, bei der Stimulierung von Phagozytose, bei der Dysregulation des Abwehr- und Immunsystem verwendet werden.

## Herstellungsbeispiele

1.) Methyl-6-O-(1-undecyl)-nonadecyloxycarbonyl-D-glucopyranosid

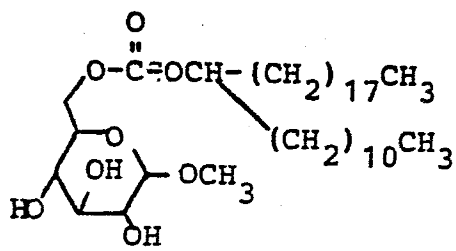

8,9 g (0,046 Mol) 1-O-Methyl-D-glucopyranosid werden mit 5,1 g Triethylamin in 150 ml Tetrahydrofuran suspendiert und bei 0°C Innentemperatur mit 23,1 g (0,046 Mol) Chlorameisensäure-(1-undecyl)-nonadecylester versetzt. Man erwärmt 3 d auf 40°C, filtriert den Feststoff ab und destilliert das Lösungsmittel im Wasserstrahlvakuum ab. Der Rückstand wird an Kieselgel chromatographiert (400 g Merck Kieselgel 60; Korngröße 0,040 - 0,063 mm; Laufmittel: Toluol: Isopropanol = 10 : 1) und man erhält 1,2 g Methyl-6-O-(1-undecyl)-nonadecyloxycarbonyl-D-glucopyranosid mit einem R.f. Wert von 0,44 (Toluol: Isopropanol = 6 : 1; Merck-DC-Alufolien; Kieselgel 60 $F_{254}$; Schichtdicke 0,2 mm).

2.) Methyl-6-O-palmitoyl-α-D-glucopyranosid

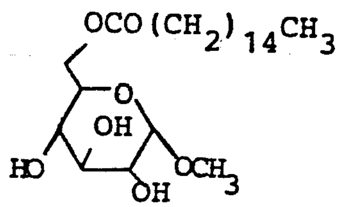

411 mg (1mmol) Methyl-2,3,4-O-tris-trimethylsilyl-α-D-glucopyranosid, 256 mg (1mmol) Palmitinsäure und 206 mg (1mmol) Dicyclohexylcarbodiimid werden in 10 ml Methylenchlorid 4 Stunden bei 20°C gerührt. Nach Abfiltrieren des Harnstoffs wird gut mit Methylenchlorid nachgewaschen und das Lösungsmittel im Wasserstrahlvakuum abdestilliert. Man erhält in quantitativer Ausbeute Methyl-2,3,4-O-tris-trimethylsilyl-6-O-palmitoyl-α-D-glucopyranosid mit einem Rf.-Wert von 0,45 (Ether : Petrolether = 1:1, Merck-Platten Art. Nr. 5719).

700 mg (1,08 mmol) dieses Produktes werden in 10 ml $CH_3OH$ heiß gelöst und mit Wasser versetzt (10 ml). Man erhitzt 4 h unter Rückfluß und entfernt dann flüchtige Produkte im Hochvakuum. Man erhält in quantitativer Ausbeute Methyl-6-O-palmitoyl-α-D-glucopyranosid mit einem Rf.-Wert von 0,19, ($CH_3OH:H_2O$ = 15 : 1)

Analog erhält man:

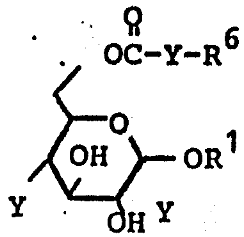

| | $R^1$ | Y | $R^6$ | Rf.-Wert* |
|---|---|---|---|---|
| 3.) | $CH_3$ | O | $-CH$ $(CH_2)_8CH_3$<br>$(CH_2)_8CH_3$ | 0,26 |
| 4.) | $CH_3$ | O | $-CH$ $(CH_2)_{16}CH_3$<br>$(CH_2)_{17}CH_3$ | |
| 5.) | $CH_3$ | O | $-CH$ $(CH_2)_{14}CH_3$<br>$(CH_2)_{17}CH_3$ | |
| 6.) | $CH_3$ | O | $-CH$ $(CH_2)_{11}$ | 0,37 |
| 7.) | $CH_3(CH_2)_{11}$ | O | $-(CH_2)_{11}CH_3$ | |
| 8.) | $CH_3(CH_2)_{11}$ | $CH_2$ | $-(CH_2)_{11}CH_3$ | |
| 9.) | $CH_3(CH_2)_{17}$ | $CH_2$ | $-(CH_2)_{10}CH_3$ | |
| 10.) | " | $CH_2$ | $-(CH_2)_{16}CH_3$ | |
| 11.) | " | NH | $-(CH_2)_{17}CH_3$ | |
| 12.) | " | S | $-(CH_2)_{11}CH_3$ | |

*) (Toluol: i-PrOH = 6:1) Merck DC-Platten Art.Nr. 5714

Patentansprüche

1. Verbindungen der Formel I

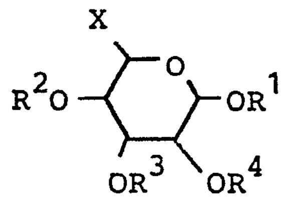

(I)

in welcher

X für Wasserstoff oder den Rest $CH_2OR^5$ steht,

$R^2$, $R^3$, $R^4$, $R^5$ gleich oder verschieden sind und für Wasserstoff oder den Rest

$$-\overset{O}{\overset{\parallel}{C}}-Y-R^6$$

stehen,

Y für Sauerstoff, Schwefel, NH, $CH_2$ steht,

$R^1$, $R^6$ gleich oder verschieden sind und für einen gegebenenfalls substituierten Kohlenwasserstoffrest mit bis zu 50 Kohlenstoffatomen steht mit der Maßgabe, daß mindestens einer der Reste $R^1$ und $R^6$ zwischen 9 und 50 Kohlenstoffatome enthält,

zur Bekämpfung von Krankheiten.

2. Verbindungen nach Anspruch 1, dadurch gekennzeichnet, daß $R^1$ und $R^6$ unabhängig voneinander für einen gegebenenfalls substituierten Alkyl- oder Alkenylrest mit 1 - 21 C-Atomen stehen, zur Bekämpfung von Krankheiten.

3. Verbindungen nach den Ansprüchen 1 oder 2, dadurch gekennzeichnet, daß X für $CH_2OR^5$ steht und $R^5$ die in Anspruch 1 angegebene, von Wasserstoff verschiedene Bedeutung hat, zur Bekämpfung von Krankheiten.

4. Verbindungen nach Anspruch 1, dadurch gekennzeichnet, daß $R^1$ und $R^6$ unabhängig voneinander für einen gegebenenfalls substituierten Alkyl- oder Alkenylrest mit 9 - 21 C-Atomen steht, zur Bekämpfung von Krankheiten.

5. Verbindungen nach Anspruch 1, dadurch gekennzeichnet, daß $R^1$ für $CH_3$, $CH_3(CH_2)_{11}$ oder $CH_3(CH_2)_{17}$ steht, Y O, $CH_2$, NH oder S bedeutet und $R^6$ für

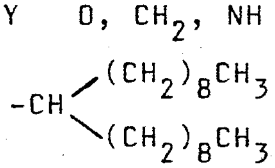 , 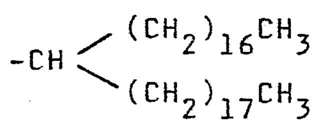 , 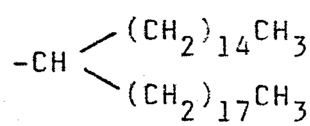 ,

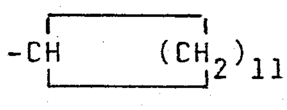, $-(CH_2)_{11}CH_3$, $-(CH_2)_{10}CH_3$, $-(CH_2)_{16}CH_3$,

$-(CH_2)_{17}CH_3$

steht.

6. Methyl-6-O-(1-undecyl)-nonadecyloxycarbonyl-D-glucopyranosid zur Bekämpfung von Krankheiten.

7. Methyl-6-O-palmitoyl-α-D-glucopyranosid zur Bekämpfung von Krankheiten.

8. Arzneimittel enthaltend mindestens eine Verbindung der Formel I

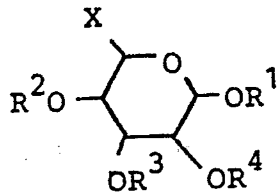

(I)

in welcher

X für Wasserstoff oder den Rest $CH_2OR^5$ steht,

$R^2$, $R^3$, $R^4$, $R^5$ gleich oder verschieden sind und für Wasserstoff oder den Rest $-\overset{O}{\overset{\|}{C}}-Y-R^6$ stehen,

Y für Sauerstoff, Schwefel, NH, $CH_2$ steht,

$R^1$, $R^6$ gleich oder verschieden sind und für einen gegebenenfalls substituierten Kohlenwasserstoffrest mit bis zu 50 Kohlenstoffatomen steht mit der Maßgabe, daß mindestens einer der Reste $R^1$ und $R^6$ zwischen 9 und 50 Kohlenstoffatome enthält.

9. Verwendung der Verbindungen der Formel I

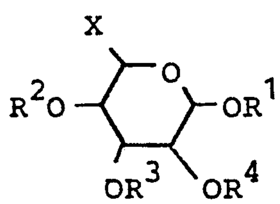

(I)

in welcher

X für Wasserstoff oder den Rest $CH_2OR^5$ steht,

$R^2$, $R^3$, $R^4$, $R^5$ gleich oder verschieden sind und für Wasserstoff oder den Rest

$-\overset{\overset{O}{\|}}{C}-Y-R^6$ stehen,

Y für Sauerstoff, Schwefel, NH, $CH_2$ steht,

$R^1$, $R^6$ gleich oder verschieden sind und für einen gegebenenfalls substituierten Kohlenwasserstoffrest mit bis zu 50 Kohlenstoffatomen steht mit der Maßgabe, daß mindestens einer der Reste $R^1$ und $R^6$ zwischen 9 und 50 Kohlenstoffatome enthält,

bei der Bekämpfung von Krankheiten.

10. Verwendung von Verbindungen der Formel I

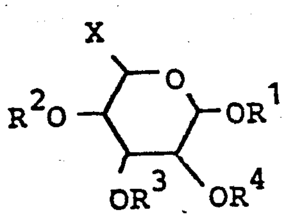

(I)

in welcher

X für Wasserstoff oder den Rest $CH_2OR^5$ steht,

$R^2$, $R^3$, $R^4$, $R^5$ gleich oder verschieden sind und für Wasserstoff oder den Rest

$-\overset{O}{\overset{\|}{C}}-Y-R^6$ stehen,

Y für Sauerstoff, Schwefel, NH, $CH_2$ steht,

zur Herstellung von Arzneimitteln.